# EUROPEAN PATENT APPLICATION

(11) **EP 2 810 692 A1**
(43) Date of publication of application: **10.12.2014**
(21) Application number: 13425081.0
(22) Date of filing: 05.06.2013
(51) Int. Cl.: A61N 2/00, A61N 2/02

(54) **Device for magnetotherapy**

(71) Applicant: Led S.p.A., 03100 Frosinone (IT)
(72) Inventor: Mauti, Paolo, 04011 Aprilia LT (IT); Intini, Lorenzo, 03100 Frosinone FR (IT); Quattrini, Valentino, 03100 Frosinone FR (IT)
(74) Representative: Papa, Elisabetta

(57) **Abstract**

A device (1) apt to perform a magnet therapy treatment of persons and animals, allowing to treat subjects in a manner compliant with a specific therapeutic protocol and without a break in the applying of the treatment itself. The device mainly comprises a main body (2) in the form of a mat or carpet incorporating a pair of magnetic field delivery plates (20A, 20B), a programmable control unit (3) and a system for detection of the presence of a subject lying on the main body (Fig. 1).

## Description

### Technical Field of the Invention

The present invention refers to a magnet therapy device, suitable to deliver a magnet therapy treatment that can be had by persons or animals, particularly pets such as dogs and cats.

### Background

In the last years the employ of magnetic fields for therapeutic purposes, both in human and animal subjects, has caught on. In particular, on the market there are magnet therapy devices usable also in a household environment and wherein the magnetic field delivering system is incorporated in a mat or in a carpet on which the subject may be positioned, e.g. seated, lying down or crouched. Such known devices typically provide a manually operable switch for initiating the treatment, i.e. the delivery of the magnetic field according to a predetermined program.

The employ of the above-introduced known devices is limited by the fact that a subject may need to leave the mat or carpet during a delivery of the treatment, which therefore is had incompletely and accordingly proves scarcely effective. Such a drawback is particularly emphasized in case of treatment delivered to children or animals, as the latter tend to move without any warning and therefore without enabling a supervising adult to understand whether and/or to what extent the treatment has been had.

In other words, in known devices the uninterrupted stay of the subject on the mat or carpet is actually a necessary condition for correctly having the treatment.

Moreover, the abovementioned drawback exposes the device to a repeated and prolonged use, and therefore to a quicker wear.

### Summary of the Invention

Therefore, the technical problem set and solved by the present invention is that of providing a magnet therapy device allowing to overcome the drawbacks mentioned above with reference to the known art.

Such a problem is solved by a device according to claim 1.

Preferred features of the present invention are the subject of the dependent claims.

The main advantage of the invention lies in the fact that the proposed device enables to treat subjects in a manner compliant with the specific therapeutic protocol prescribed, without a break in the applying of the treatment itself, but allowing anyhow to the subject a temporary going off from the device during magnetic field delivery.

Hence, the device proves particularly effective and advantageous for the treatment of children or animals.

Moreover, the proposed configuration also preserves the device itself from unnecessary prolonged and repeated uses.

Other advantages, features and the operation steps of the present invention will be made apparent in the following detailed description of some embodiments thereof, given by way of example and not for limitative purposes.

### Brief description of the figures

Reference will be made to the figures of the annexed drawings, wherein:
■ Figure 1 shows an exploded view of a preferred embodiment of the magnet therapy device according to the present invention;
■ Figure 2 shows a perspective and partially sectional view of the device of Figure 1;
■ Figure 3 shows a cross-sectional view of the device of Figure 1, taken along line A-A of Figure 2;
■ Figure 4 shows a perspective view of the device of Figure 1 in use;
■ Figure 5 shows a schematic block diagram depiction of the device of Figure 1; and
■ Figure 6 shows a possible exemplary electronic scheme of embodiment of a control unit of the device of Figure 1 and components associated thereto.

### Detailed description of preferred embodiments

Initially referring to Figures 1 to 3, a magnet therapy device according to a preferred embodiment of the invention is generally denoted by 1.

The device 1 comprises a substantially mat, or carpet, shaped main body 2. In particular, in the present example the main body 2 has prevalent extension in a *xy* plane, i.e. in width and length, and reduced thickness in the *z* direction.

The main body 2 is shaped overall so as to be able to receive thereon a subject, be it an animal or a human being, in need of a magnet therapy treatment and who may be positioned seated, lying down, crouched, etc. In particular, as mentioned above the main body 2 may be substantially mat, or carpet, shaped according to any shape and size suitable to ensure the subject's treatment, in particular of the entire body thereof or even of a specific body district only.

The main body 2 provides a substantially sandwich-like structure, i.e. formed by two external layers, respectively denoted by 21 and 22, and by a intermediate layer 20 suitable to the emission of electromagnetic waves - and generally to the delivery of a magnetic field - for the treatment of a human or animal subject.

Preferably, the external layers 21 and 22 are at least partly made of an insulating material. Preferably, they are at least partly made of natural or synthetic fabric.

In the present embodiment, the intermediate layer 20 is made of a pair of strips or plates 20A and 20B arranged side-by-side on the above-mentioned transversal *xy* plane and separated by a longitudinal gap or space (in direction *x* in the example considered), so as to be galvanically insulated. The arrangement is such that between such strips 20A and 20B, in use, magnetic field lines be set up, and that said strips may act as armatures for measuring the capacitance that may be set up therebetween, during a step of measuring for the detection of the presence of animate bodies on the device 1, step that will be described shortly.

The electromagnetic field generated by the intermediate layer 20 has frequency suitable to application in the medical field and dependent on the type of treatment carried out. In a preferred embodiment, the intermediate layer 20 is apt to generate, when in use, a magnetic field of a frequency of about 25 MHz, preferably with a modulating frequency of 500 to 5000 Hz and strength comprised in a range of 2-20 uT for high frequency (10 - 25 MHz) and 5-100 mT for any low-frequency (12.5-1000 Hz) application.

Preferably, the intermediate layer 20 is made of an electrically conductive material, preferably copper.

Preferably, such intermediate layer is made in the form of conductive fabric.

The device 1 moreover comprises a control unit, generally denoted by 3. The control unit 3 comprises storage means for storing one or more magnetic field delivery programs, preferably differentiated in terms of duration.

The control unit 3, in the present example, is housed at the main body 2, and in particular inside a pocket-like seat 25 obtained in the top layer 21 of the main body itself. The pocket-like seat 25 provides closure means 26, e.g. of clip or button type, selectively openable to access the control unit 3.

Through the pocket-like seat 25 a means for manually turning on or activating the device 1 is accessible, in particular a pushbutton 30.

On the control unit 3, means for signaling a state (e.g., ON/OFF, active or in standby, as will be mentioned below) may also be provided, in particular LEDs or other luminous means, preferably in view.

In the present example, the intermediate layer 20 is electrically connected to the control unit 3 by a pair of connection contacts 41 and 42, each connected, at one end, to a respective strip 20A, 20B by a respective terminal 411, 421. Preferably, the latter is substantially pincer, or clamp, shaped.

The other end of the contacts 41 and 42 may also be associated to a power supply source, directly or by the control unit 3. In particular, in the present embodiment it is envisaged that the power supply be obtained by batteries housed at or near the control unit 3, or anyhow in the pocket-like seat 25 or in a similar seat obtained on the main body 2.

In the present example, the contacts 41 and 42 are operatively connected to the control unit 3 and realize means apt to detect when a subject is positioned on the main body 2.

In particular, the contacts 41 and 42 with the terminals 411 and 421 realize means for measuring the capacitive coupling between the two strips 20A and 20B. In fact, the overall configuration of the device 1 is such that when a person or an animal is positioned on the main body 2 a change in capacitive coupling is produced with respect to a basic measurement detected in the absence of a subject.

Capacitive coupling measurement can be performed by the so-called CVD (*Capacitive Voltage Divider*) technique (as known, e.g., from: Application Note Microchip AN1298 - Capacitive Touch Using Only an ADC ("CVD")).

As mentioned, the control unit 3 is operatively connected to the detecting means considered herein, and configured to produce an automatic activation of the magnetic field delivery layer 20, i.e. of the plates 20A and 20B, in response to a person, or animal, detection signal provided by the detecting means itself. Analogously, the control unit 3 is configured so as to cause the interruption of magnetic field delivery by the intermediate layer 20 when the detection signal does not indicate the presence of a subject on the main body 2.

Moreover, the control unit 3 is configured so that the device 1, once turned on, e.g. by means of the pushbutton 30, may assume a rest or standby state, in which said magnet therapy treatment is not delivered, and an active state, in which the intermediate layer 20 delivers the magnetic field, where the transit from said rest state to said active state, and vice versa, is caused by the detection, or non-detection, of the subject's presence on the main body 2.

The device 1 - and in particular the programming/configuration of its control unit 3, of the detecting means and the plates 20A and 20B - enables the operation modes illustrated hereinafter, also referring to Figure 4.

The device 1 can be turned on by means of the pushbutton 30.

At turning-on, without a subject positioned on the main body 2, the device 1 performs a basic capacitive coupling detection between plates 20A and 20B, thereby performing a setting or calibrating step. The device 1 may be configured to emit a signal, e.g. acoustic, when this step has ended and the device 1 is therefore ready for use.

At turning-on, by means of a selector (not depicted) a desired treatment program, i.e. of delivery of magnetic field by means of the intermediate layer 20, may also be selected.

The device 1 assumes the so-called standby state. In said state, the control unit 3 and/or the detecting means are configured so as to perform periodical detecting of the presence of the subject on the main body 2, i.e., in the present example, periodical measurements of the capacitive coupling between plates 20A and 20B.

When a subject positions him/her/itself on the main body 2, the subsequent measurement indicates a capacitive coupling, possibly according to a preset threshold, different to the value metered in the calibrating stage. The control unit 3 then causes the transit of the device 1 from the standby state to the active one, initiating delivery of the magnetic field of treatment.

The control unit 3 is moreover configured so that, in said active state, the delivery of a magnetic field be periodically interrupted, for a very limited time, and a measurement of capacitive coupling between the two plates 20A and 20B be performed. If a capacitive coupling value indicative of a subject's absence from the main body 2 is detected, the magnetic field delivery, i.e. the treatment, is not carried on further, and the device returns to the stand-by state.

When a subject's presence on the main body 2 is detected again, the control unit 3 again causes transit from the standby state to the active one, resuming treatment delivery according to the program already started and for the remaining time.

These operations, i.e. the transit from the standby state to the active one and vice versa, can be repeated plural times during the delivery of a same treatment.

Preferably, to safeguard device integrity, the control unit 3 may be programmed for an automatic turning off of the device itself after a certain predetermined time in the ON state and/or for the interruption of magnetic field delivery after a cumulative maximum time has elapsed, e.g. daily.

The block diagram in Figure 5 exemplifies, in a non-limiting way, some of the above-introduced components. Therein, "microcontroller" corresponds to the above-mentioned control unit. Moreover, the analog switch provided therein consists in an electronic semiconductor switch apt to separate the emitting portion of the circuitry from the measuring one (high impedance).

By way of a mere example, a possible electronic principle diagram for the control unit 3 is reported in Figure 6. The microcontroller software implements the above-described calibration, measurement and delivery procedures.

The calibration and measurement functions are very similar and consist in the following steps:
- bringing the analog switch into high impedance;
- loading the parasitic capacitance present in the microcontroller ADC by switching, via a software, the internal "sample and hold" module on the AN0 channel (permanently connected to supply voltage);
- then, selecting the measurement channel AN1 (permanently connected to one of the two plates or strips of the intermediate layer 20);
- digitally measuring, by the microcontroller ADC module, the voltage value;
- closing the analog switch, thereby letting the radiofrequency signal through.

By this method, with the increase of the capacitive coupling between the two plates or strips of the intermediate layer 20, the voltage value measured by the microcontroller ADC decreases.

From the turning-on of the device, the first measurement is reserved to the calibration stage, which stores a voltage value. The subsequent measurement stages compare the new values measured with the initial one, thereby initiating the treatment in case of subject's presence on the mat.

Variant embodiments may provide a location of the control unit 3 remote of the main body 2, and possibly a wireless-type connection between the control unit itself and the other components of the device 1 itself.

Moreover, variant embodiments may provide a different implementation of the above-mentioned detecting means, e.g. in the form of pressure, proximity, or optic sensors or transducers or other types of magnetic means, operatively connected to the control unit.

Moreover, further variants may provide a different realization of the electromagnetic wave-emitting means, possibly in the form of two parts galvanically insulated therebetween.

Moreover, the aforesaid strips of conductive material may be replaced by plates, armatures in general or other elements, anyhow suitable to the delivery of a magnetic field / to the emission of electromagnetic waves.

Again, an option of a merely manual activation of treatment delivery may be provided, e.g. by the same above-introduced pushbutton or a separate means.

Moreover, it has to be noted that the abovementioned control unit and detecting means may be made in the form of a single component or structural and/or functional block.

The present invention has hereto been described with reference to preferred embodiments thereof. It is understood that other embodiments might exist, all falling within the concept of the same invention, as defined by the protective scope of the claims hereinafter.

## Claims

1. A magnet therapy device (1) suitable to perform a magnet therapy treatment on a human or animal subject, which device (1) comprises:
- a substantially mat, or carpet, shaped main body (2), having a substantially sandwich-like structure formed by two external layers (21, 22), preferably of natural or synthetic fabric, and by a intermediate layer (20) for magnetic field delivery, preferably of conductive fabric, which intermediate layer (20) can be activated to produce a magnetic field that, in use, traverses a subject positioned on the main body itself (2);
- detecting means (411, 421), apt to detect when a subject is positioned on said main body (2); and
- a control unit (3), operatively connected to said detecting means (411, 421) and configured to produce an automatic activation of said layer (20) for magnetic field delivery in response to a subject detection signal provided by said detecting means (411, 421) and/or an automatic interruption of said magnetic field delivery by said intermediate layer (20) in response to a subject non-detection signal provided by said detecting means (411, 421).

2. The device (1) according to claim 1, wherein said control unit (3) and/or said detecting means (411, 421) are configured so as to perform periodical detecting of the subject's presence on said main body (2).

3. The device (1) according to any one of the preceding claims, which is configured so as to be able to assume a rest or standby state, wherein said magnetic field is not delivered, and an active state, wherein said intermediate layer (20) delivers said magnetic field, and wherein the configuration is such that the transit from said rest state to said active state, and vice versa, is automatically caused by the detection, or non-detection, of the subject's presence on said main body (2).

4. The device (1) according to any one of the preceding claims, wherein said magnetic field delivery layer (20) comprises a pair of elements (20A, 20B), preferably in the form of strips or plates, galvanically insulated therebetween and configured so that therebetween, in use, magnetic field lines are set up.

5. The device (1) according to any one of the preceding claims, wherein said intermediate layer (20) is made of two parts (20A, 20B) galvanically insulated therebetween, preferably separated from each other, and said detecting means (411, 421) comprises means for measuring a capacitive coupling between said two parts, the overall configuration being such that when a subject is positioned on said main body (2) a variation of said capacitive coupling is produced.

6. The device (1) according to the preceding claim, wherein said control unit (3) is configured so as to periodically interrupt, in use, magnetic field delivery by said intermediate layer (20) to command the performing of said measurement of capacitive coupling between said two parts (20A, 20B) of said intermediate layer (20).

7. The device (1) according to any one of the preceding claims, wherein said control unit (3) comprises storage means for storing one or more magnetic field delivery programs, preferably differentiated in terms of duration.

8. The device (1) according to the preceding claim, wherein said control unit (3) is configured so as to enable the interruption of magnetic field delivery according to a predetermined program and the subsequent resumption of said delivery for the remaining time provided by the program itself.

9. The device (1) according to any one of the preceding claims, comprising means (30) for the manual activation of said layer (20) for magnetic field delivery.

10. The device (1) according to any one of the preceding claims, comprising means for power-supplying said delivery layer (20), preferably arranged at or near said control unit (3).

11. The device (1) according to any one of the preceding claims, wherein said control unit (3) is housed at said main body (2), preferably at a pocket-like seat (25) obtained in said main body (2).

12. The device (1) according to any one of the preceding claims, wherein said intermediate layer (20) is apt to generate, in use, a magnetic field of frequency comprised in a range of about 12.5 - 25 MHz and/or of strength comprised in a range of about 2-20 microTesla.

13. The device (1) according to any one of the preceding claims, wherein said external layers (21, 22) are made of an insulating material.

14. The device (1) according to any one of the preceding claims, wherein said intermediate layer (20) is at least partly made of an electrically conductive material.
